# EUROPÄISCHE PATENTANMELDUNG

(11) **EP 0 774 459 A1**
(43) Veröffentlichungstag der Anmeldung: **21.05.1997**
(21) Anmeldenummer: 96117606.2
(22) Anmeldetag: 04.11.1996
(51) Int. Cl.: C07C 311/08, C07D 213/65, A61K 31/155, A61K 31/44

(54) **Sulfonylamino-substituierte Benzoylguanidine, Verfahren zu ihrer Herstellung, ihre Verwendung als Medikament oder Diagnostikum sowie sie enthaltendes Medikament**

(30) Priorität: 14.11.1995 DE 19542306
(71) Anmelder: HOECHST AKTIENGESELLSCHAFT, 65929 Frankfurt am Main (DE)
(72) Erfinder: Kleemann, Heinz-Werner, Dr., 65474 Bischofsheim (DE); Brendel, Joachim, Dr., 61118 Bad Vilbel (DE); Schwark, Jan-Robert, Dr., 65929 Frankfurt (DE); Weichert, Andreas, Dr., 63329 Egelsbach (DE); Lang, Hans Jochen, Dr., 65719 Hofheim (DE); Albus, Udo, Dr., 61197 Florstadt (DE); Scholz, Wolfgang, Dr., 65760 Eschborn (DE)

(57) **Zusammenfassung**

Benzoylguanidine der Formel I 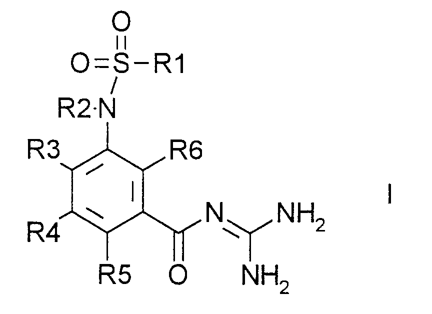 sind hervorragend wirksame antiarrhythmische Arzneimittel mit cardioprotektiver Komponente und sind zur Infarktprophylaxe und Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern.

## Beschreibung

Die Erfindung betrifft Benzoylguanidine der Formel I 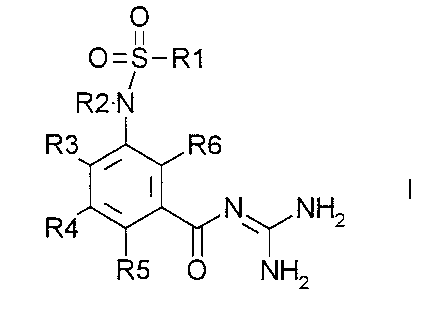 worin bedeuten:
- R(1): Alkyl mit 1, 2, 3, 4, 5, 6, 7 oder 8 C-Atomen oder NR(7)R(8);
- R(7) und R(8): unabhängig voneinander Wasserstoff oder Alkyl mit 1, 2, 3, 4, 5, 6, 7 oder 8 C-Atomen
- R(2): Wasserstoff, Alkyl mit 1, 2, 3, 4, 5, 6, 7 oder 8 C-Atomen oder -SO₂R(9)
- R(9): unabhängig wie R(1) definiert;
- R(3): Wasserstoff, -SR(25), -OR(25), -NR(25)R(26) oder -CR(25)R(26)R(27);
- R(25): Wasserstoff, Alkyl mit 1, 2, 3, 4, 5, 6, 7 oder 8 C-Atomen, oder Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
- R(25): -(C₁-C₉)-Heteroaryl,
das unsubstituiert oder substituiert ist mit 1 - 3 Substituenten ausgewählt aus der Gruppe bestehend aus der Gruppe F, Cl, CF₃, CH₃, Methoxy, Hydroxy, Amino, Methylamino und Dimethylamino;
- R(26) und R(27): unabhängig voneinander wie R(25) definiert oder Wasserstoff oder Alkyl mit 1, 2, 3, 4, 5, 6, 7 oder 8 C-Atomen;
- R(4): Wasserstoff, F, Cl, Br, I, OH, -C≡N, CF₃, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
- m: Null, 1 oder 2;
- R(14): -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16); R(15) und R(16)
unabhängig voneinander Wasserstoff oder -CH₃;
- R(5) und R(6): unabhängig voneinander Wasserstoff, Alkyl mit 1, 2, 3 oder 4 C-Atomen, F, Cl, -OR(32), -NR(33)R(34) oder CF₃;
- R(32), R(33) und R(34): unabhängig voneinander Wasserstoff oder Alkyl mit 1, 2, 3 oder 4 C-Atomen;
sowie deren pharmazeutisch verträgliche Salze.

Bevorzugt sind Verbindungen der Formel I, in denen bedeuten:
- R(1): Alkyl mit 1, 2, 3 oder 4 C-Atomen oder NR(7)R(8);
- R(7) und R(8): unabhängig voneinander Wasserstoff oder Alkyl mit 1, 2, 3 oder 4 C-Atomen;
- R(2): Wasserstoff, Alkyl mit 1, 2, 3 oder 4 C-Atomen oder -SO₂R(9)
- R(9): unabhängig wie R(1) definiert;
- R(3): Wasserstoff, -SR(25), -OR(25), -NR(25)R(26) oder -CR(25)R(26)R(27);
- R(25): Wasserstoff, Alkyl mit 1, 2, 3 oder 4 C-Atomen oder Phenyl,
das unsubstituiert oder substituiert ist mit 1 - 2 Substituenten ausgewählt aus der Gruppe bestehend aus F, Cl, CF₃, CH₃, Methoxy und Dimethylamino;
oder
- R(25): -(C₁-C₉)-Heteroaryl,
das unsubstituiert oder substituiert ist mit 1 - 2 Substituenten ausgewählt aus der Gruppe bestehend aus F, Cl, CF₃, CH₃, Methoxy und Dimethylamino;
- R(26) und R(27): unabhängig voneinander Wasserstoff oder Alkyl mit 1, 2, 3 oder 4 C-Atomen;
- R(4): Wasserstoff, F, Cl, Br, I, OH, CF₃, Alkyl mit 1, 2, 3 oder 4 C-Atomen, Alkenyl mit 2, 3 oder 4 C-Atomen oder (CH₂)ₘR(14);
- m: Null, 1 oder 2;
- R(14): -(C₃-C₆)-Cycloalkyl oder Phenyl, welches nicht substituiert oder substituiert ist mit 1 - 2 Substituenten ausgewählt aus der Gruppe bestehend aus F, Cl, -CF₃, Methyl und Methoxy;
- R(5) und R(6): unabhängig voneinander Wasserstoff, Alkyl mit 1, 2 oder 3 C-Atomen, F, Cl, -OR(32), -NR(33)R(34) oder CF₃;
- R(32), R(33) und R(34): unabhängig voneinander Wasserstoff oder Methyl;
sowie deren pharmazeutisch verträgliche Salze.

Besonders bevorzugt sind Verbindungen der Formel I, in denen bedeuten:
- R(1): Methyl oder Dimethylamino;
- R(2): Wasserstoff, Methyl, -SO₂CH₃ oder -SO₂N(CH₃)₂;
- R(3): Wasserstoff, -SR(25), -OR(25), -NR(25)R(26) oder -CR(25)R(26)R(27);
- R(25): Wasserstoff, Alkyl mit 1, 2 oder 3 C-Atomen oder Phenyl,
das unsubstituiert oder substituiert ist mit einem Substituenten ausgewählt aus der Gruppe bestehend aus F, Cl, CF₃ und CH₃;
oder
- R(25): -(C₁-C₉)-Heteroaryl,
das unsubstituiert oder substituiert ist mit einem Substituenten ausgewählt aus der Gruppe bestehend aus F, Cl, CF₃ und CH₃;
- R(26) und R(27): unabhängig voneinander Wasserstoff oder Methyl;
- R(4): Wasserstoff, F, Cl, OH, CF₃ oder Alkyl mit 1, 2, 3 oder 4 C-Atomen;
- R(5) und R(6): unabhängig voneinander Wasserstoff, Alkyl mit 1, 2 oder 3 C-Atomen, F, Cl, -OR(32), -NR(33)R(34) oder CF₃;
- R(32), R(33) und R(34): unabhängig voneinander Wasserstoff oder Methyl;
sowie deren pharmazeutisch verträgliche Salze.

Ganz besonders bevorzugt sind Verbindungen der Formel I, in denen bedeuten:
- R(1): Methyl oder Dimethylamino;
- R(2): Wasserstoff;
- R(3): Wasserstoff, -OR(25) oder -CR(25)R(26)R(27);
- R(25): Wasserstoff, Alkyl mit 1, 2 oder 3 C-Atomen oder Phenyl,
das unsubstituiert oder substituiert ist mit einem Substituenten ausgewählt aus der Gruppe bestehend aus F, Cl, CF₃ und CH₃; oder
- R(25): -(C₁-C₉)-Heteroaryl,
das unsubstituiert oder substituiert ist mit einem Substituenten ausgewählt aus der Gruppe bestehend aus F, Cl, CF₃ und CH₃;
- R(26) und R(27): unabhängig voneinander Wasserstoff oder Methyl;
- R(4): Wasserstoff, OH, CF₃ oder Alkyl mit 1, 2, 3 oder 4 C-Atomen;
- R(5) und R(6): unabhängig voneinander Wasserstoff, Alkyl mit 1, 2 oder 3 C-Atomen, F, Cl oder CF₃;
sowie deren pharmazeutisch verträgliche Salze.

Die bezeichneten Alkylreste können sowohl geradkettig wie verzweigt vorliegen.

Unter (C₁₋C₉)-Heteroaryl werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Ganz besonders geeignet sind die Heterocyclen Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Indolyl, Chinolyl und Isochinolyl.

Enthält einer der Substituenten R(1) bis R(6) ein oder mehrere Asymmetriezentren, so können diese unabhängig voneinander sowohl S als auch R konfiguriert sein. Die Verbindungen können als optische Isomere, als Diastereomere, als Racemate oder als Gemische derselben vorliegen.

Die Erfindung betrifft weiterhin ein Verfahren zur Herstellung der Verbindungen I, dadurch gekennzeichnet, daß man Verbindungen der Formel II 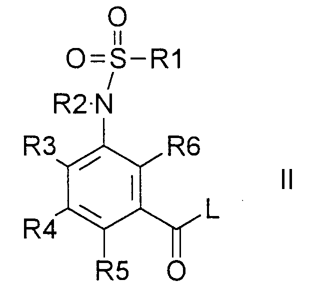 worin R(1) bis R(6) die oben angegebenen Bedeutungen haben und L für eine leicht nucleophil substituierbare Fluchtgruppe steht, mit Guanidin umsetzt. Die aktivierten Säurederivate der Formel II, worin L eine Alkoxy-, vorzugsweise eine Methoxygruppe oder Phenoxygruppe, eine Phenylthio-, Methylthio-, 2-Pyridylthiogruppe, oder einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann. Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1,351-367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluoroborat ("TOTU") (Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der allgemeinen Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF zwischen 20°C und Siedetemperatur dieser Lösungsmittel bewährt. Bei den meisten Umsetzungen von Verbindungen II mit salzfreien Guanidin wurde vorteilhaft in inerten Lösungsmitteln wie THF, Dimethoxyethan, Dioxan oder Isopropanol gearbeitet. Aber auch Wasser kann als Lösungsmittel dienen.

Wenn L = Cl bedeutet, arbeitet man vorteilhaft unter Zusatz eines Säurefängers, z.B. in Form von überschüssigen Guanidin zur Abbindung der Halogenwasserstoffsäure.

Die Einführung der im Phenylteil mit Schwefel-, Sauerstoff- oder Stickstoffnucleophilen substituierten Benzolsulfonamid-Derivate gelingt durch literaturbekannte Methoden der nucleophilen Substitution an Derivaten der 3-Nitrobenzoesäure. Als Abgangsgruppe am Benzoesäurederivat haben sich bei dieser Substitution Halogenide und Trifluormethansulfonate bewährt. Man arbeitet vorteilhaft in einem dipolar aprotischen Lösungsmittel, wie DMF oder TMU, bei einer Temperatur von 0°C bis zum Siedepunkt des Lösungsmittels, bevorzugt von 80°C bis zum Siedepunkt des Lösungsmittels. Als Säurefänger dient vorteilhaft ein Alkali- oder Erdalkalisalz mit einem Anion hoher Basizität und geringer Nucleophilie, zum Beispiel K₂CO₃ oder Cs₂CO₃.

Die Reduktion der Nitroverbindung zum Anilin können die hinlänglich bekannten Standardverfahren angewendet werden. Bewährt hat sich zum Beispiel die Reduktion mit Eisenpulver in Methanol und konzentrierter wäßriger HCl-Lösung.

Überraschenderweise kann die Nitrogruppe auch mit Natriummethansulfinat direkt in die Methansulfonylamino-Gruppe überführt werden. Dazu wird die Nitroverbindung zusammen mit Natriummethansulfinat in einem dipolar aprotischen Lösungsmittel, wie zum Beispiel DMF, TMU oder NMP bei einer Temperatur zwischen RT und dem Siedepunkt des Lösungsmittels, bevorzugt von 80°C bis 160°C umgesetzt.

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Benzoylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Ascorbate, Lactate, Sulfate, Citrate, Tartrate, Acetate, Phosphate, Methylsulfonate, p-Toluolsulfonate.

In der US-Patentschrift 5 091 394 (HOE 89/F 288) und in der europäischen Offenlegungsschrift 0 556 674 (HOE 92/F 034) werden Benzoylguanidine beschrieben, in welchen aber die Substituenten nicht die nach der vorliegenden Erfindung beanspruchten Bedeutungen haben. Es sind keine Benzolsulfonimidamid-Derivate beschrieben. Außerdem läßt die Wasserlöslichkeit dieser bekannten Benzoylguanidine zu wünschen übrig. Bekannt sind daraus auch Benzoylguanidine mit Sulfamoyl-Substituenten R₂N-SO₂-; jedoch sind keine Verbindungen der erfindungsgemäßen Art bekannt oder nahegelegt, die eine Aminosulfonyl-Gruppe -NR(2)SO₂-R(1) tragen.

Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ -Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den meisten bekannten Verbindungen weisen die Verbindungen nach der Erfindung eine signifikant verbesserte Wasserlöslichkeit auf. Daher sind sie wesentlich besser für i.v.-Applikation geeignet.

Die erfindungsgemäßen Verbindungen zeichnen sich gegenüber den bekannten gut wasserlöslichen Verbindungen durch ihre bessere Bioverfügbarkeit und Pharmakokinetik aus.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg Körpergewicht, vorzugsweise mindestens 0,01 mg/kg Körpergewicht, bis höchstens 10 mg/kg Körpergewicht, vorzugsweise bis höchstens 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

### Liste der Abkürzungen:

- AIBN: α,α-Azo-bis-isobutyronitril
- Bn: Benzyl
- Brine: gesättigte wäßrige NaCl-Lösung
- CH₂Cl₂: Dichlormethan
- DCI: Desorption-Chemical Ionisation
- DIP: Diisopropylether
- DMA: Dimethylacetamid
- DME: Dimethoxyethan
- DMF: N,N-Dimethylformamid
- EE: Ethylacetat (EtOAc)
- EI: electron impact
- eq: Äquivalent
- ES: Elektrospray-Ionisation
- Et: Ethyl
- FAB: Fast Atom Bombardment
- HEP: n-Heptan
- HOAc: Essigsäure
- Me: Methyl
- MeOH: Methanol
- mp: Schmelzpunkt
- MTB: Methyltertiärbutylether
- NBS: N-Bromsuccinimid
- NMP: N-Methylpyrrolidon
- RT: Raumtemperatur
- THF: Tetrahydrofuran
- TMU: N,N,N',N'-Tetramethylharnstoff
- Tol: Toluol
- ZNS: Zentralnervensystem

### Experimenteller Teil

### Allgemeine Vorschrift zur Herstellung von Benzoyl-guanidinen (I) Variante A: aus Benzoesäuren (II, L = OH)

0,01 M des Benzoesäurederivates der Formel II löst bzw. suspendiert man in 60m wasserfreiem THF und versetzt sodann mit 1,78g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCl auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I) Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Allgemeine Vorschrift zur Herstellung von Benzoyl-guanidinen (I) Variante C: Aus Benzoesäure-alkylestern (II, L = O-Alkyl), Guanidin-Freisetzung in situ

25 mmol Kalium-t-butylat werden in 100 ml DMF (wasserfrei) gelöst und mit 30 mmol Guanidin-hydrochlorid versetzt. 1 h wird bei RT gerührt, anschließend 5 mmol des Benzoesäure-alkylesters der Formel II zugegeben und bei RT (typische Reaktionszeit 1 - 24 h) oder bei 80°C (typische Reaktionszeit 10 Minuten bis 4 h) bis zum vollständigen Umsatz (Dünnschichtkontrolle) gerührt. Das Reaktionsgemisch wird auf 500 ml Wasser gegossen, mit verdünnter wäßriger HCl-Lösung auf pH = 8 - 9 gestellt, 1 h gerührt und abgesaugt. Das Produkt wird im Vakuum getrocknet und falls nötig an Kieselgel mit einem geeigneten Laufmittel, z. B. EE/MeOH 5 : 1 chromatographiert. (Salzbildung vergleiche Variante A)

### Beispiel 1: 4-Isopropyl-3-methylsulfonylamino-benzoylguanidin, Hydrochlorid

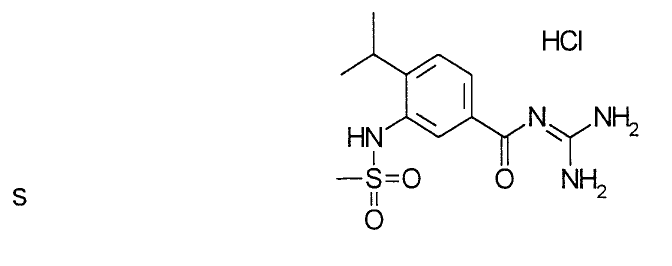

a) 4-Isopropyl-3-methylsulfonylamino-benzoesäuremethylester
   1.46 g 4-Isopropyl-3-nitro-benzoesäuremethylester und 1.34 g Natriummethansulfinat werden in DMF 4 h unter Rückfluß erhitzt. Nach Abkühlung auf RT wird das Reaktionsgemisch zu 100 ml gesättigter wäßriger NaHCO₃-Lösung gegeben und 3 mal mit je 100 ml EE extrahiert. Über Na₂SO₄ wird getrocknet und das Solvens im Vakuum entfernt. Chromatographie des Rückstandes an Kieselgel mit DIP liefert 400 mg eines farblosen Öls.
   R_{f} (DIP) = 0.14 MS (DCI) : 272 (M + H)⁺
b) 4-Isopropyl-3-methylsulfonylamino-benzoylguanidin
   380 mg 4-Isopropyl-3-methylsulfonylamino-benzoesäuremethylester werden zusammen mit 414 mg Guanidin in 10 ml *i*-Propanol nach der allgemeinen Vorschrift zur Herstellung von Benzoyl-guanidinen, Variante B umgesetzt. Nach Chromatographie an Kieselgel mit EE/MeOH 5:1 erhält man 110 mg eines amorphen Feststoffs, der mit wäßriger HCl-Lösung in das Hydrochlorid überführt wird. mp (Hydrochlorid) 224 ^{o}C
   R_{f} (EE/MeOH 5:1) = 0.39 MS (ES) : 299 (M + H)⁺

### Beispiel 2: 3-Methylsulfonylamino-4-(3-pyridyloxy)-benzoylguanidin

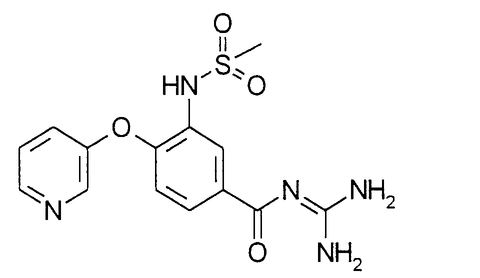

a. 3-Nitro-4-(3-pyridyloxy)-benzoesäuremethylester
   30 g 3-Hydroxypyridin, 68 g 4-Chlor-3-nitro-benzoesäuremethylester und 87 g K₂CO₃ werden in 500 ml NMP 1 h bei 120 ^{o}C gerührt. Nach Abkühlung auf RT wird auf 3 l Wasser gegossen und 3 mal mit je 1 l CH₂Cl₂ extrahiert. Die organische Phase wird anschließend 2 mal mit je 1 l Wasser gewaschen, über Na₂SO₄ getrocknet und das Solvens im Vakuum entfernt. Man erhält 71 g eines teilweise kristallisierenden, braunen Öls, das ohne weitere Reinigung verwendet wird.
   R_{f} (EE) = 0.39 MS (EI) : 274 (M)⁺
b. 3-Amino-4-(3-pyridyloxy)-benzoesäuremethylester
   71 g 3-Nitro-4-(3-pyridyloxy)-benzoesäuremethylester und 52 g Eisenpulver werden in 500 ml MeOH gerührt und bei RT langsam mit 500 ml gesättigter wäßriger HCl-Lösung versetzt. 2 h wird bei RT nachgerührt, dann die flüchtigen Bestandteile im Vakuum entfernt. Der Rückstand wird mit 300 ml gesättigter wäßriger Na₂CO₃-Lösung (pH = 11) ausgerührt und der Niederschlag abgesaugt. Anschließend wird dieser Niederschlag 2 mal mit je 500 ml EE ausgekocht und das Filtrat 2 mal mit je 500 ml EE extrahiert. Die vereinigte EE-Phase wird 2 mal mit je 500 ml Wasser gewaschen, über Na₂SO₄ getrocknet und das Solvens im Vakuum entfernt. Man erhält 30 g gelbbrauner Kristalle, mp 101^{o}C.
   R_{f} (MTB) = 0.32 MS (EI) : 244 (M)⁺
c. 3-Bis-(methylsulfonyl)amino-4-(3-pyridyloxy)-benzoesäuremethylester
   1 g 3-Amino-4-(3-pyridyloxy)-benzoesäuremethylester und 1 ml Triethylamin werden in 30 ml CH₂Cl₂ gelöst und langsam 400 µl Methansulfonylchlorid zugetropft. Über Nacht wird bei RT gerührt, anschließend mit 100 ml CH₂Cl₂ verdünnt und 1 mal mit 100 ml gesättigter wäßriger Na₂CO₃-Lösung gewaschen. Über Na₂SO₄ wird getrocknet und das Solvens im Vakuum entfernt. Man erhält 1.5 g eines hellgelben Öls. R_{f} (MTB) = 0.28 MS (EI) : 400 (M)⁺
d. 3-Methylsulfonylamino-4-(3-pyridyloxy)-benzoesäure*-i-*propylester
   1.5 g 3-Bis-(methylsulfonyl)amino-4-(3-pyridyloxy)-benzoesäuremethylester und 1.1 g Guanidin werden in 10 ml *i-*Propanol gelöst und 2 h lang unter Rückfluß erhitzt. Das Solvens wird im Vakuum entfernt, 200 ml Wasser zugegeben, mit wäßriger HCl-Lösung auf pH = 7 gestellt und 3 mal mit je 100 ml EE extrahiert. Über Na₂SO₄ wird getrocknet und das Solvens im Vakuum entfernt. Chromatographie an Kieselgel mit EE/MeOH 5:1 liefert 570 mg eines farblosen Öls.
   R_{f} (EE/MeOH 5:1) = 0.46 MS (ES) : 351 (M + H)⁺
e. 3-Methylsulfonylamino-4-(3-pyridyloxy)-benzoylguanidin
   570 mg 3-Methylsulfonylamino-4-(3-pyridyloxy)-benzoesäure*-i-*propylester und 600 mg Guanidin werden in 2 ml i-Propanol nach der allgemeinen Vorschrift zur Herstellung von Benzoyl-guanidinen, Variante B umgesetzt (Reaktionszeit 3 h). Nach Chromatographie an Kieselgel mit EE/MeOH 5 : 1 erhält man 160 mg eines amorphen Feststoffs. Eine Probe wird mit wäßriger HCl-Lösung in das Hydrochlorid überführt, das aufgrund seiner außergewöhnlich hygroskopischen Eigenschaften ebenfalls keinen definierten Schmelzpunkt liefert. R_{f} (EE/MeOH 5:1) = 0.13 MS (ES) : 350 (M + H)⁺

### Beispiel 3: 2-Methyl-4-isopropyl-5-methylsulfonylamino-benzoylguanidin

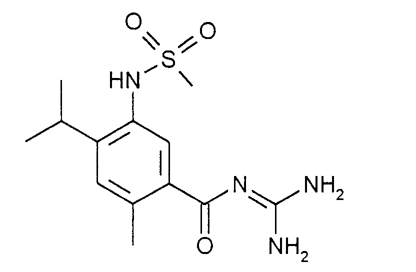

a) 2-Methyl-4-brom-5-nitro-benzoesäure
   200 ml H₂SO₄ werden zu 200 ml einer 65% wäßrigen HNO₃-Lösung langsam zugetropft. Dann werden bei 0 ^{o}C 30 g 4-Brom-2-methyl-benzoesäure zugegeben und 3 h bei dieser Temperatur gerührt. Das Reaktionsgemisch wird auf 800 g Eis gegossen, 1 h nachgerührt, das Produkt abfiltriert und getrocknet. Man erhält 33 g eines farblosen Feststoffs, mp 164°C (enthält kleine Menge an 3-Nitro-Isomer).
   MS (DCI) : 260 (M + H)⁺
b) 2-Methyl-4-brom-5-nitro-benzoesäuremethylester
   33 g 2-Methyl-4-brom-5-nitro-benzoesäure werden in 500 ml MeOH gelöst und langsam mit 26.7 ml SOCl₂ versetzt. 3 h wird unter Rückfluß gekocht, anschließend die flüchtigen Bestandteile im Vakuum entfernt. Der Rückstand wird mit 100 ml Toluol versetzt und erneut die flüchtigen Bestandteile im Vakuum entfernt. Man erhält 37 g farbloser Kristalle, die zur Entfernung des 3-Nitro-Isomeren aus DIP umkristallisiert werden. Daraus erhält man 17 g Produkt, mp 104°C.
   R_{f} (EE/HEP 1:4) = 0.26 MS (DCI) : 274 (M + H)⁺
c) 2-Methyl-4-brom-5-amino-benzoesäuremethylester
   13 g 2-Methyl-4-brom-5-nitro-benzoesäuremethylester werden in 200 ml MeOH gelöst und 11.5 g Eisenpulver zugegeben. Anschließend werden langsam 200 ml einer gesättigten wäßrigen HCl-Lösung zugetropft. 2 h wird bei RT gerührt, die flüchtigen Bestandteile im Vakuum entfernt, mit 400 ml Na₂CO₃ und 200 ml EE aufgenommen und abfiltriert. Der Rückstand wird 3 mal mit je 600 ml EE jeweils 15 Minuten gekocht, die wäßrige Phase 2 mal mit je 200 ml EE extrahiert. Die EE-Phasen werden vereinigt, über Na₂SO₄ getrocknet und das Solvens im Vakuum entfernt. Man erhält 7.6 g eines dunklen Öls.
   R_{f} (EE/HEP 1:4) = 0.14 MS (DCI) : 244 (M + H)⁺
d) 2-Methyl-4-brom-5-(di-methylsulfonyl)amino-benzoesäuremethylester
   7.6 g 2-Methyl-4-brom-5-amino-benzoesäuremethylester werden in 300 ml CH₂Cl₂ gelöst, 21.5 ml Triethylamin zugegeben, bei 0°C 5.1 ml Methansulfonylchlorid zugetropft und 2 h bei RT gerührt. Das Reaktionsgemisch wird in 300 ml einer gesättigten wäßrigen NaHCO₃-Lösung eingerührt, die CH₂Cl₂-Phase abgetrennt und noch 3 mal mit je 200 ml EE extrahiert. Die vereinigten organischen Phasen werden über Na₂SO₄ getrocknet und die Solventien im Vakuum entfernt. Man erhält 10.9 g gelber Kristalle, mp 221°C.
   R_{f} (DIP) = 0.42 MS (DCI) : 399 (M)⁺
e) 2-Methyl-4-brom-5-methylsulfonylamino-benzoesäuremethylester
   2.9 g 2-Methyl-4-brom-5-(di-methylsulfonyl)amino-benzoesäuremethylester und 0.81 g Kalium-t-butylat werden in 100 ml MeOH 4 h bei RT gerührt. Das Reaktionsgemisch wird auf 250 ml einer gesättigten wäßrigen NaHSO₄-Lösung und 250 ml Wasser gegossen, das MeOH im Vakuum entfernt und der Feststoff abgesaugt. Im Vakuum wird getrocknet und man erhält 2.0 g farbloser Kristalle, mp 138°C.
   R_{f} (DIP) = 0.15 MS (DCI) : 321 (M)⁺
f) 2-Methyl-4-isopropyl-5-methylsulfonylamino-benzoesäuremethylester
   28 ml einer 2 M Lösung von Isopropylmagnesiumchlorid in THF werden zu 134 ml einer 0.5 M Lösung von ZnCl₂ in THF getropft und 6 h bei 55 - 60°C gerührt. Anschließend wird bei RT 2.0 g 2-Methyl-4-brom-5-methylsulfonylamino-benzoesäuremethylester, 230 mg CuI und 522 mg [1,1'-Bis-(diphenylphosphino)-ferrocen]-palladium(II)-chlorid zugegeben und 18 h bei RT gerührt. Das Reaktionsgemisch wird mit 500 ml EE verdünnt und der Niederschlag abfiltriert. 2 mal wird mit je 200 ml 5% wäßriger NaHSO₄-Lösung und 2 mal mit je 200 ml gesättigter wäßriger NaCl-Lösung gewaschen, über Na₂SO₄ getrocknet und die Solventien im Vakuum entfernt. Chromatographie an Kieselgel mit DIP liefert 1.1 g eines farblosen Öls.
   R_{f} (DIP) = 0.21 MS (DCI) : 286 (M + H)⁺
g) 2-Methyl-4-isopropyl-5-methylsulfonylamino-benzoylguanidin
   550 mg 2-Methyl-4-isopropyl-5-methylsulfonylamino-benzoesäuremethylester werden nach der allgemeinen Vorschrift zur Herstellung von Benzoyl-guanidinen (I), Variante C guanyliert (Reaktionszeit 3 h bei 80 ^{o}C). Man erhält 270 mg eines amorphen weißen Pulvers.
   R_{f} (EE/MeOH 5:1) = 0.41 MS (ES) : 313 (M + H)⁺

### Pharmakologische Daten:

Inhibition des Na⁺/H⁺ -Exchangers von Kaninchenerythrocyten Weiße Neuseeland-Kaninchen (Ivanovas) erhielten eine Standard-Diät mit 2% Cholesterin für sechs Wochen, um den Na⁺/H⁺ -Austausch zu aktivieren und so den Na⁺ -Influx in die Erythrocyten via Na⁺/H⁺ -Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺ -Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺ -Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

### Inhibition des Na⁺/H⁺ -Exchangers:

| Beispiel | IC₅₀ [ µmol/l] |
|---|---|
| 1 | 0.3 |
| 2 | 0.74 |

## Patentansprüche

1. Benzoylguanidine der Formel I 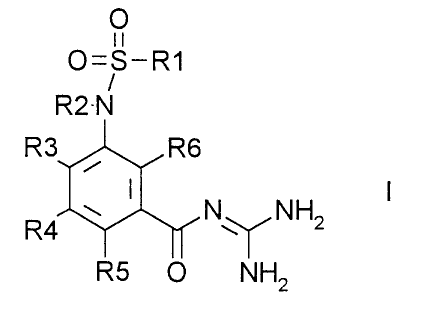 worin bedeuten:
R(1) Alkyl mit 1, 2, 3, 4, 5, 6, 7 oder 8 C-Atomen oder NR(7)R(8);
R(7) und R(8)
unabhängig voneinander Wasserstoff oder Alkyl mit 1, 2, 3, 4, 5, 6, 7 oder 8 C-Atomen
R(2) Wasserstoff, Alkyl mit 1, 2, 3, 4, 5, 6, 7 oder 8 C-Atomen oder -SO₂R(9)
R(9) unabhängig wie R(1) definiert;
R(3) Wasserstoff, -SR(25), -OR(25), -NR(25)R(26) oder -CR(25)R(26)R(27);
R(25) Wasserstoff, Alkyl mit 1, 2, 3, 4, 5, 6, 7 oder 8 C-Atomen, oder Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(25)
-(C₁-C₉)-Heteroaryl,
das unsubstituiert oder substituiert ist mit 1 - 3 Substituenten ausgewählt aus der Gruppe bestehend aus der Gruppe F, Cl, CF₃, CH₃, Methoxy, Hydroxy, Amino, Methylamino und Dimethylamino;
R(26) und R(27)
unabhängig voneinander wie R(25) definiert oder Wasserstoff oder Alkyl mit 1, 2, 3, 4, 5, 6, 7 oder 8 C-Atomen;
R(4) Wasserstoff, F, Cl, Br, I, OH, -C≡N, CF₃, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
unabhängig voneinander Wasserstoff oder -CH₃;
R(5) und R(6)
unabhängig voneinander Wasserstoff, Alkyl mit 1, 2, 3 oder 4 C-Atomen, F, Cl, -OR(32), -NR(33)R(34) oder CF₃;
R(32), R(33) und R(34)
unabhängig voneinander Wasserstoff oder Alkyl mit 1, 2, 3 oder 4 C-Atomen;
sowie deren pharmazeutisch verträgliche Salze.

2. Verbindung der Formel I nach Anspruch 1, in der bedeuten:
R(1) Alkyl mit 1, 2, 3 oder 4 C-Atomen oder NR(7)R(8);
R(7) und R(8)
unabhängig voneinander Wasserstoff oder Alkyl mit 1, 2, 3 oder 4 C-Atomen;
R(2) Wasserstoff, Alkyl mit 1, 2, 3 oder 4 C-Atomen oder -SO₂R(9)
R(9) unabhängig wie R(1) definiert;
R(3) Wasserstoff, -SR(25), -OR(25), -NR(25)R(26) oder -CR(25)R(26)R(27);
R(25) Wasserstoff, Alkyl mit 1, 2, 3 oder 4 C-Atomen oder Phenyl,
das unsubstituiert oder substituiert ist mit 1 - 2 Substituenten ausgewählt aus der Gruppe bestehend aus F, Cl, CF₃, CH₃, Methoxy und Dimethylamino;
oder
R(25) -(C₁-C₉)-Heteroaryl,
das unsubstituiert oder substituiert ist mit 1 - 2 Substituenten ausgewählt aus der Gruppe bestehend aus F, Cl, CF₃, CH₃, Methoxy und Dimethylamino;
R(26) und R(27)
unabhängig voneinander Wasserstoff oder Alkyl mit 1, 2, 3 oder 4 C-Atomen;
R(4) Wasserstoff, F, Cl, Br, I, OH, CF₃, Alkyl mit 1, 2, 3 oder 4 C-Atomen, Alkenyl mit 2, 3 oder 4 C-Atomen oder -(CH₂)ₘR(14);
m Null, 1 oder 2;
R(14) -(C₃-C₆)-Cycloalkyl oder Phenyl,
welches nicht substituiert oder substituiert ist mit 1 - 2 Substituenten ausgewählt aus der Gruppe bestehend aus F, Cl, -CF₃, Methyl und Methoxy;
R(5) und R(6)
unabhängig voneinander Wasserstoff, Alkyl mit 1, 2 oder 3 C-Atomen, F, Cl, -OR(32), -NR(33)R(34) oder CF₃;
R(32), R(33) und R(34)
unabhängig voneinander Wasserstoff oder Methyl.

3. Verbindung der Formel I nach Anspruch 1 oder 2, in der bedeuten:
R(1) Methyl oder Dimethylamino;
R(2) Wasserstoff, Methyl, -SO₂CH₃ oder -SO₂N(CH₃)₂;
R(3) Wasserstoff, -SR(25), -OR(25), -NR(25)R(26) oder -CR(25)R(26)R(27);
R(25) Wasserstoff, Alkyl mit 1, 2 oder 3 C-Atomen oder Phenyl,
das unsubstituiert oder substituiert ist mit einem Substituenten ausgewählt aus der Gruppe bestehend aus F,
Cl, CF₃ und CH₃;
oder
R(25) -(C₁-C₉)-Heteroaryl,
das unsubstituiert oder substituiert ist mit einem Substituenten ausgewählt aus der Gruppe bestehend aus F, Cl, CF₃ und CH₃;
R(26) und R(27)
unabhängig voneinander Wasserstoff oder Methyl;
R(4) Wasserstoff, F, Cl, OH, CF₃ oder Alkyl mit 1, 2, 3 oder 4 C-Atomen;
R(5) und R(6)
unabhängig voneinander Wasserstoff, Alkyl mit 1, 2 oder 3 C-Atomen, F, Cl, -OR(32), -NR(33)R(34) oder CF₃;
R(32), R(33) und R(34)
unabhängig voneinander Wasserstoff oder Methyl.

4. Verbindung der Formel I nach Ansprüchen 1, 2 oder 3, in der bedeuten:
R(1) Methyl oder Dimethylamino;
R(2) Wasserstoff;
R(3) Wasserstoff, -OR(25) oder -CR(25)R(26)R(27);
R(25) Wasserstoff, Alkyl mit 1, 2 oder 3 C-Atomen oder Phenyl,
das unsubstituiert oder substituiert ist mit einem Substituenten ausgewählt aus der Gruppe bestehend aus F, Cl, CF₃ und CH₃;
oder
R(25) -(C₁-C₉)-Heteroaryl,
das unsubstituiert oder substituiert ist mit einem Substituenten ausgewählt aus der Gruppe bestehend aus F, Cl, CF₃ und CH₃;
R(26) und R(27)
unabhängig voneinander Wasserstoff oder Methyl;
R(4) Wasserstoff, OH, CF₃ oder Alkyl mit 1, 2, 3 oder 4 C-Atomen;
R(5) und R(6)
unabhängig voneinander Wasserstoff, Alkyl mit 1, 2 oder 3 C-Atomen, F, Cl oder CF₃.

5. Verfahren zur Herstellung einer Verbindung I nach Anspruch 1, dadurch gekennzeichnet, daß man
eine Verbindung der Formel II 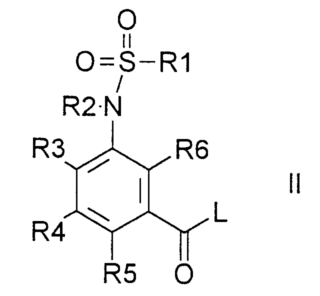 worin R(1) bis R(6) die in Anspruch 1 angegebenen Bedeutungen haben und L für eine leicht nucleophil substituierbare Fluchtgruppe steht, mit Guanidin umsetzt.

6. Verwendung einer Verbindung I nach Anspruch 1 zur Herstellung eines Medikaments zu Behandlung von Arrhythmien.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch I zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen diabetische Spätkomplikationen, Krebserkrankungen, fibrotische Erkrankungen wie Lungenfibrose, Leberfibrose oder Nierenfibrose, Prostatahyperplasie.

16. Heilmittel, enthaltend eine wirksame Menge einer Verbindung I nach einem oder mehreren der Ansprüche 1 bis 4.
